(19) Europäisches Patentamt
European Patent Office
Office européen des brevets

(11) **EP 2 465 503 A1**

(12) **EUROPEAN PATENT APPLICATION**
published in accordance with Art. 153(4) EPC

(43) Date of publication:
**20.06.2012 Bulletin 2012/25**

(51) Int Cl.:
**A61K 31/495** (2006.01) **A61K 9/08** (2006.01)
**A61P 37/08** (2006.01) **A61P 17/00** (2006.01)

(21) Application number: **10807854.4**

(22) Date of filing: **11.08.2010**

(86) International application number:
**PCT/CN2010/001221**

(87) International publication number:
**WO 2011/017909 (17.02.2011 Gazette 2011/07)**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **12.08.2009 CN 200910165840
31.10.2009 CN 200910211361**

(71) Applicant: **Lunan Better Pharmaceutical Co., Ltd.
Shandong 276006 (CN)**

(72) Inventor: **ZHAO, Zhiquan
Shandong 276006 (CN)**

(74) Representative: **Høiberg A/S
St. Kongensgade 59 A
1264 Copenhagen K (DK)**

(54) **PHARMACEUTICAL SOLUTION OF CETIRIZINE HYDROCHLORIDE**

(57)     A pharmaceutical solution comprising cetirizine hydrochloride, an alcohol compound with less than 3 carbon atoms, a preservative and urea is disclosed. The cetirizine hydrochloride is in racemic form or in levo form.

EP 2 465 503 A1

Printed by Jouve, 75001 PARIS (FR)

**Description**

Technical Field

The present invention relates to a pharmaceutical solution of cetirizine hydrochloride, and specifically to a pharmaceutical solution of racemic cetirizine hydrochloride and L-cetirizine hydrochloride.

Background Art

In recent years, with the increase of environmental pollution, the abuse of chemicals and the increase of the stimulation of external harmful substances, the incidence of skin allergy is increasing year by year. When entering human body, an external allergen would activate T and B lymphatic systems, so as to induce the degranulation of mast cells, and release allergic inflammatory mediators such as histamine, bradykinin and the like, resulting in allergic response which manifests as different kinds of allergic diseases, such as allergic rhinitis, urticaria, skin irritation, itching, red spots, allergen-induced asthma, etc. Such diseases, although not directly threatening the life, seriously affect the patient's quality of life due to their complicated pathogenesis and high incidence. Therefore, the development of the drug for the treatment of allergic diseases is very important.

At present, the medicines for the clinical treatment of allergic diseases are mainly second-generation anti-histamines agents, among which cetirizine has relatively strong activity and good anti-allergic and anti-inflammatory effects. Cetirizine hydrochloride was firstly developed by UCB Company, Belgium and came into the market in 1987. Most of the current cetirizine hydrochloride formulations are conventional oral preparations, such as tablets, syrups, etc, and are mainly absorbed through the gastrointestinal tract and then distributed throughout the dermal tissue in the skin by blood circulation, so as to act as an antagonist of histamine, an inflammatory mediator, and to further treat the systemic allergic skin inflammations. The distribution of blood flow in the skin is relatively low, which results in that the distribution of cetirizine in the tissues is insufficient for the treatment of allergic skin diseases and dermatitis. Oral administration in large doses is not an ideal administration route, as it may render serious systemic adverse reactions in respect of skin diseases.

In view of the adverse reactions resulted from oral preparations of cetirizine hydrochloride for the treatment of allergic diseases, Lunan Pharmaceutical Co., Ltd. firstly discloses ordinary liniment, liposome liniment, aerosol and ointment of cetirizine hydrochloride (CN1634063A, pp 9-13), in which the ordinary liniment of cetirizine hydrochloride consists of cetirizine hydrochloride, ethanol, propylene glycol and distilled water (or phosphate buffered saline (PBS)); the liposome liniment of cetirizine hydrochloride consists of cetirizine hydrochloride, lecithin, cholesterol, ceramide or octadecylamine, vitamin E, menthol, and phosphate buffered saline (PBS); the ointment of cetirizine hydrochloride consists of cetirizine hydrochloride and one or more of carboxymethyl cellulose, glycerin, ethyl paraben, urea, tween-80 and triethanolamine; and the aerosol of cetirizine hydrochloride consists of cetirizine hydrochloride, ethanol, urea, dichlorodifluoromethane, vitamin C and distilled water. The development and the preparation of topical formulations provide a new administration route, which greatly reduces the adverse reactions.

L-cetirizine is the R-enantiomer of cetirizine, and the affinity between the L-cetirizine and histamine H1 receptor is three times as much as that between racemic cetirizine and histamine H1 receptor. L-cetirizine hydrochloride is generally adopted in clinic, and oral preparation thereof is applied to alleviate the allergy symptoms of allergic diseases. Patent applications CN1813743A and CN1957942A disclose topical formulations of L-cetirizine hydrochloride, including liniment, aerosol, spray, ointment and transdermal patch. CN1957942A (pp 7-9) discloses liniment consisting of L-cetirizine hydrochloride, ethanol, propylene glycol and water; aerosol consisting of L-cetirizine hydrochloride, ethanol, urea, dichlorodifluoromethane, vitamin C and water; and spray consisting of L-cetirizine hydrochloride, ethanol, dimethyl sulfoxide, vitamin C and water.

Symptoms such as swelling and itching caused by mosquitoes stings are the common conditions in the summer and the autumn. Upon piercing into the skin, the mosquitoes secrete formic acid through their mouthpart, resulting in the feel of stinging. There may be erythema, papules or wheal in the stung site with a dark-red blood spot in the center of the injured sites, which can not completely fade when press-diagnosed. It is characterized by a pale circle around the pain points. The degrees of redness and itching varies, in which some people have no symptoms after being stung, and some ones only feel mild itching and slight pain. Some ones with allergy, however, may show a significant swelling, and even large areas of stasis spot, accompanied by intense itching and burning feeling.

Currently, there are some mistakes in the treatment of mosquito stings, in which the floral water is widely used. Camphor and other ingredients in the floral water cannot help to alleviate local inflammation; instead, they cause an allergic reaction, resulting in contact dermatitis. Therefore, it is necessary to develop a medicine with significant therapeutic effects and weaker stimulations. An article in Journal for Beneficial Readings: Drug Information & Medical Advices (2009, Issue 6, page 63) teaches not to abuse irritating medicines when stung by mosquitoes; the medicines should be carefully used under the guidance of a physician. Although the floral water has the effects of cooling and alleviating itching, it does not have therapeutic efficacy. As mentioned in JIANGSU Labour Protection (2007, Issue 8, page 45), for some people with sensitive skin, camphor and other ingredients in the floral water cannot help to alleviate local inflammation; instead, they lead to allergic reactions, resulting in contact dermatitis.

Racemic cetirizine hydrochloride may be orally administered for treatment of immediate urticaria and immediate papilla and itching caused by mosquito stings. However, its effect in practice is not very good, and thus is not widely used in daily life. It is believed that the main reason is either unsatisfying results, or the orally administered medicine need a longer time to exert the effects, while the itching and tingling, although intensive in a short time after sting, would spontaneously ease very soon.

Chinese Patent Application CN1634063A generally discloses a topical preparation of cetirizine hydrochloride, but fails to disclose a formulation with ideal antipruritic effects. Upon the application of the liniment and the spray containing L-cetirizine hydrochloride disclosed on pages 7-9 of CN1957942A in the treatment of mosquito stings, the inventors found that although they have certain therapeutic effect, they tend to cause adverse reactions such as strong skin irritation. It is believed that the main reason thereof is that most patients having less tolerance to mosquito stings have tender skins, while the solutions of the above mentioned medicines contain ethanol, which has large irritation to the skin.

Summary of the invention

In order to increase the therapeutic effects of topical preparations of cetirizine hydrochloride, to reduce the skin irritancy of the topical preparations of cetirizine hydrochloride and to provide a useful medicine for treating swelling and itching caused by mosquito stings, the present invention optimizes the formula in accordance with the physicochemical properties of cetirizine hydrochloride, screens suitable adjuvants by experiments, and provides a pharmaceutical solution of cetirizine hydrochloride. The pharmaceutical solution of cetirizine hydrochloride according to the present invention has low skin irritancy and rapid transdermal absorption, and is therefore suitable for use in the patients with different ages and with skin allergy. Moreover, the pharmaceutical solution may be prepared with a simple process with fewer raw materials and lower cost, and thus may have a wide application.

In one aspect, the present invention provides a pharmaceutical solution of cetirizine hydrochloride, wherein cetirizine hydrochloride is racemic cetirizine hydrochloride or L-cetirizine hydrochloride. The pharmaceutical solution comprises the following components:

| | |
|---|---|
| Cetirizine hydrochloride | 0.1~2 parts by weight; |
| Alcohol having 3 or less carbon atoms | 1-50 parts by weight; |
| Preservative | 0.01~0.2 parts by weight; |
| Urea | 0.02~20 parts by weight; and |
| Inorganic salt | 0~5 parts by weight. |

In one embodiment, the cetirizine hydrochloride in the medical composition is L-cetirizine hydrochloride, and the pharmaceutical solution of L-cetirizine hydrochloride comprises the following components:

| | |
|---|---|
| Cetirizine hydrochloride | 0.2~2 parts by weight; |
| Alcohol having 3 or less carbon atoms | 10-40 parts by weight; |
| Preservative | 0.01~0.2 parts by weight; |
| Urea | 1~5 parts by weight; and |
| Inorganic salt | 0~5 parts by weight. |

More preferably, the pharmaceutical solution of L-cetirizine hydrochloride comprises the following components:

| | |
|---|---|
| L-cetirizine hydrochloride | 0.5~1.5 parts by weight; |
| Alcohol having 3 or less carbon atoms | 15-40 parts by weight; |
| Preservative | 0.05~0.2 parts by weight; |
| Urea | 1~3 parts by weight; and |
| Inorganic salt | 0~5 parts by weight. |

Further preferably, the pharmaceutical solution of L-cetirizine hydrochloride comprises the following components:

| L-cetirizine hydrochloride | 1 parts by weight; |
|---|---|
| Alcohol having 3 or less carbon atoms | 15-40 parts by weight; |
| Preservative | 0.1 parts by weight; |
| Urea | 2 parts by weight; and |
| Inorganic salt | 0.5~1.5 parts by weight. |

Preferably, in the pharmaceutical solution of L-cetirizine hydrochloride, the alcohol having 3 or less carbon atoms may be propanediol, glycerin, propanol, ethanol or any combination thereof; more preferably, the alcohol having 3 or less carbon atoms may be glycerin or 1,2-propanediol; even more preferably, the alcohol having 3 or less carbon atoms may be glycerin. Said preservative may be one or two selected from benzalkonium bromide, chlorhexidine acetate, sodium benzoate and sorbic acid; more preferably, the preservative may be benzalkonium bromide or chlorhexidine acetate; even more preferably, the preservative may be benzalkonium bromide. Said inorganic salt may be one or more selected from sodium chloride, potassium chloride, sodium sulfate, potassium sulfate, calcium chloride and sodium bicarbonate; more preferably the inorganic salt may be sodium chloride.

More preferably, the pharmaceutical solution of L-cetirizine hydrochloride according to the present invention may further comprise a flavoring agent which may be selected from orange peel oil, anise oil, citron oil, citron tincture, a flagrant, cinnamon water, cinnamon oil, banana essence, orange essence, lemon essence and apple essence.

Preferably, the pharmaceutical solution of L-cetirizine hydrochloride according to the present invention has a pH value of 5.0-7.0, more preferably a pH value of 6.0.

The pharmaceutical solution of L-cetirizine hydrochloride according to the present invention may be further formulated into liniment, drop, aerosol or spray by conventional process.

According to one embodiment of the present invention, the pharmaceutical solution of cetirizine hydrochloride according to the present invention is a pharmaceutical solution of racemic cetirizine hydrochloride comprising the following components:

| Cetirizine hydrochloride | 0.1~2 parts by weight; |
|---|---|
| Alcohol having 3 or less carbon atoms | 1-50 parts by weight; |
| Preservative | 0.02~0.2 parts by weight; |
| Urea | 0.02~20 parts by weight; and |
| Inorganic salt | 0.01~2 parts by weight, |

wherein the alcohol having 3 or less carbon atoms is glycerin; the preservative is benzalkonium bromide, and the inorganic salt is sodium chloride. The combination of glycerin as a wetting agent, urea as a transdermal enhancer and benzalkonium bromide as a preservative facilitates skin absorption by local administration and could effectively increase the local concentration of the medicine in the allergic skin, and the plasma protein binding rate of cetirizine reaches 93% or above. Before entering the blood, cetirizine locally absorbed by the skin is present in a free state in the skin tissue, thus can exhibit the anti-allergic effect more effectively.

Preferably, the pharmaceutical solution of racemic cetirizine hydrochloride comprises the following components:

| Cetirizine hydrochloride | 0.5~1.5 parts by weight; |
|---|---|
| Glycerin | 5~40 parts by weight; |
| Urea | 1~6 parts by weight; |
| Benzalkonium bromide | 0.02~0.15 parts by weight; and |
| Sodium chloride | 0.5~1.5 parts by weight. |

More preferably, the pharmaceutical solution of racemic cetirizine hydrochloride comprises the following components:

| Cetirizine hydrochloride | 1 part by weight; |
|---|---|
| Glycerin | 35~40 parts by weight; |
| Urea | 1~3 parts by weight; |
| benzalkonium bromide | 0.08~0.12 parts by weight; and |
| Sodium chloride | 0.5~1.5 parts by weight. |

Even more preferably, the pharmaceutical solution of racemic cetirizine hydrochloride comprises the following components:

| | |
|---|---|
| Cetirizine hydrochloride | 1 part by weight; |
| Glycerin | 37.8 parts by weight; |
| Urea | 2 parts by weight; |
| Benzalkonium bromide | 0.1 parts by weight; and |
| Sodium chloride | 0.9 parts by weight. |

Preferably, the pharmaceutical solution of racemic cetirizine hydrochloride according to the present invention has a pH value of 4.0-7.5, more preferably a pH value of 5.0-7.0, and even more preferably a pH value of 6.0.

According to the present invention, the substance for adjusting the pH value of the pharmaceutical solution of racemic cetirizine hydrochloride may be basic potassium salt, basic sodium salt, basic phosphate or any combination thereof. Preferably, the substance for adjusting the pH value of the pharmaceutical solution of racemic cetirizine hydrochloride may be one or more of sodium hydroxide, sodium carbonate, sodium bicarbonate, sodium dihydrogen phosphate or disodium hydrogen phosphate; more preferably, the substance for adjusting the pH value may be sodium hydroxide or sodium carbonate; even more preferably, the substance for adjusting the pH value is sodium hydroxide.

Both the pharmaceutical solution of racemic cetirizine hydrochloride and the pharmaceutical solution of L-cetirizine hydrochloride comprise an appropriate amount of water. The amount of water depends on the desired concentration of cetirizine hydrochloride in the pharmaceutical solution. Specifically, the pharmaceutical solution of racemic cetirizine hydrochloride consists of the five components (racemic cetirizine hydrochloride, glycerin, urea, benzalkonium bromide and sodium chloride) according to the above ratio and a certain volume (such as 100 ml) of water. The ratio of the five components (cetirizine hydrochloride, glycerin, urea, benzalkonium bromide and sodium chloride) in the pharmaceutical solution of racemic cetirizine hydrochloride may also be represented as a weight ratio. Preferably, in the pharmaceutical solution of racemic cetirizine hydrochloride, the weight ratio of the five components (cetirizine hydrochloride, glycerin, urea, benzalkonium bromide and sodium chloride) is cetirizine hydrochloride 0.5-1.5 g, glycerin 5-40 g, urea 1-6 g, benzalkonium bromide 0.02-0.15 g and sodium chloride 0.5-1.5 g. The pharmaceutical solution of racemic cetirizine hydrochloride consists of the five components (racemic cetirizine hydrochloride, glycerin, urea, benzalkonium bromide and sodium chloride) with the above ratio and a certain volume (such as 100 ml) of water. Even more preferably, in the pharmaceutical solution of racemic cetirizine hydrochloride, the weight ratio of the five components (cetirizine hydrochloride, glycerin, urea, benzalkonium bromide and sodium chloride) is: cetirizine hydrochloride 1 g, glycerin 37.8 g, urea 2 g, benzalkonium bromide 0.1 g and sodium chloride 0.9 g. The pharmaceutical solution of racemic cetirizine hydrochloride consists of the five components (racemic cetirizine hydrochloride, glycerin, urea, benzalkonium bromide and sodium chloride) with the above ratio above and a certain volume (such as 100 ml) of water.

The pharmaceutical solution of racemic cetirizine hydrochloride according to the present invention may be prepared by conventional methods. Specifically, racemic cetirizine hydrochloride is dissolved in an appropriate amount of distilled water under ambient temperature and pressure. Prescription amounts of Glycerin, urea, benzalkonium bromide and sodium chloride are then added into the mixture above. The pH value is then adjusted to 4.0~7.5 with one or more of sodium hydroxide, sodium carbonate, sodium bicarbonate, sodium dihydrogen phosphate and disodium hydrogen phosphate, and distilled water is added to make up to a certain volume (such as 100 ml). Alternatively, the pharmaceutical solution of racemic cetirizine hydrochloride may be prepared by mixing the solutions of racemic cetirizine hydrochloride, glycerin, urea, benzalkonium bromide or sodium chloride.

According to the study of the pharmaceutical solutions of racemic cetirizine hydrochloride or L-cetirizine hydrochloride in their antipruritic effects on 4-aminopyridine (4-AP) induced licking response in mice models, and its anti-inflammatory effect on the ear-swelling in IgE induced skin immediate-phase (IP) or late phase (LP) allergic reaction in mice models as described in Examples 28~32 of the present invention, the pharmaceutical solutions of racemic cetirizine hydrochloride according to the present invention showed more significant effects as compared to topical preparations of the pharmaceutical solutions of racemic cetirizine hydrochloride known in the art; and the pharmaceutical solutions of L-cetirizine hydrochloride according to the present invention showed more significant antipruritic and anti-inflammatory effects as compared to the spray and the liniment of L-cetirizine hydrochloride known in the art.

The pharmaceutical solution of racemic cetirizine hydrochloride provided by the present invention can be used for treating local swelling and itching caused by mosquito stings. In Example 33, the pharmaceutical solutions of racemic cetirizine hydrochloride provided by the present invention (10 mg/ml and 5 mg/ml), essential balm and cetirizine hydrochloride tablets were used in the treatment of 200 volunteers stung by mosquitoes. Clinical results showed that, the pharmaceutical solution of cetirizine hydrochloride provided by the present invention had a curing efficacy of at least 90% on the skin swelling and itching caused by mosquito stings. For the pharmaceutical solutions of cetirizine hydrochloride, the average onset times for the high dose group and the low dose group were 6 min and 4 min, respectively.

The application of the pharmaceutical solution of racemic cetirizine hydrochloride provided by the present invention does not cause any aggravation or other adverse reactions. The essential balm group (positive control group) had a curing efficacy of 86% and an average onset time of 15 min for the skin swelling and itching caused by mosquito stings, and there were 6 cases of adverse reactions. This indicates that the pharmaceutical solution of racemic cetirizine hydrochloride had good therapeutic effects on the skin swelling and itching caused by mosquito stings, and had less irritation and quicker action than essential balm. In this clinical example, the group treated with cetirizine hydrochloride tablets was set as a control. Regarding the treatment of skin swelling and itching caused by mosquito stings, there was significant difference between the group treated with cetirizine hydrochloride tablets and the group treated with the pharmaceutical solution of racemic cetirizine hydrochloride ($p < 0.05$). This indicates that the pharmaceutical solution of racemic cetirizine hydrochloride provided by the present invention had better therapeutic efficiency than the tablet of racemic cetirizine hydrochloride for the treatment of skin swelling and itching caused by mosquito stings, and therefore can be used as a topical preparation for treating skin swelling and itching caused by mosquito stings.

The pharmaceutical solution of L-cetirizine hydrochloride provided by the present invention can be used for treating skin swelling and itching caused by mosquito stings. In Example 34, the pharmaceutical solutions of L-cetirizine hydrochloride provided by the present invention (10 mg/ml and 12 mg/ml), cetirizine hydrochloride spray, liniment and essential balm were used as positive controls in the treatment of 300 volunteers stung by mosquitoes. According to the clinic results, the pharmaceutical solution of L-cetirizine hydrochloride provided by the present invention had an efficacy of at least 90% on the skin swelling and itching caused by mosquito stings. The group treated with the pharmaceutical solution of L-cetirizine hydrochloride showed significant difference, as compared to the essential balm group, the aerosol group and the liniment group regarding the therapeutic efficacy, the average onset time and adverse reactions ($p < 0.05$).

Specific Modes for Carrying out the Invention

The following examples are described for further illustrating rather than limiting the present application.

Part I: pharmaceutical solution of racemic cetirizine hydrochloride

Example 1

| | |
|---|---|
| Cetirizine hydrochloride | 1 g |
| Glycerin | 10 g |
| Urea | 0.2 g |
| Benzalkonium bromide | 0.2 g |
| Sodium chloride | 0.1 g |
| Distilled water | appropriate amount |

Adding distilled water to make up to 100 ml, and adjusting pH value to 7.5 with an appropriate amount of 10% sodium hydroxide solution at the same time.

1 g racemic cetirizine hydrochloride was dissolved in an appropriate amount of distilled water under ambient temperature and pressure. Respective amounts of the components according to the above formula, i.e., 10 g glycerin, 0.2 g urea, 0.2 g benzalkonium bromide and 0.1 g sodium chloride, were then added. Finally, distilled water was added to make up to 100 ml and the resulted solution was uniformly mixed. Meanwhile, an appropriate amount of 10% sodium hydroxide solution was added to adjust pH to 7.5.

Example 2

| | |
|---|---|
| Cetirizine hydrochloride | 0.5 g |
| Glycerin | 5 g |
| Urea | 1g |
| Benzalkonium bromide | 0.1 |
| Sodium chloride | 0.9 g |
| Distilled water | appropriate amount |

Adding distilled water to make up to 100 ml, and adjusting pH value to 6.5 with an appropriate amount of 10% sodium carbonate solution at the same time.

0.5 g racemic cetirizine hydrochloride was dissolved in an appropriate amount of distilled water under ambient temperature and pressure. Respective amounts of the components according to the above formula, i.e., 5 g glycerin, 1 g urea, 0.1 g benzalkonium bromide and 0.9 g sodium chloride, were then added. Finally, distilled water was added to make up to 100 ml and the resulted solution was uniformly mixed. Meanwhile, an appropriate amount of 10% sodium carbonate solution was added to adjust pH to 6.5.

Example 3

| | |
|---|---|
| Cetirizine hydrochloride | 1 g |
| Glycerin | 37.8 g |
| Urea | 2 g |
| 5% Benzalkonium bromide solution | 2 ml |
| Sodium chloride | 0.9 g |
| Distilled water | appropriate amount |

Adding distilled water to make up to 100 ml, and adjusting pH value to 6.0 with an appropriate amount of 10% sodium hydroxide solution at the same time.

1 g racemic cetirizine hydrochloride was dissolved in an appropriate amount of distilled water under ambient temperature and pressure. Respective amounts of the components according to the above formula, i.e., 37.8 g glycerin, 2 g urea, 2 ml 5% benzalkonium bromide solution and 0.9 g sodium chloride, were then added. Finally, distilled water was added to make up to 100 ml and the resulted solution was uniformly mixed. Meanwhile, an appropriate amount of 10% sodium hydroxide solution was added to adjust pH to 6.0.

Example 4

| | |
|---|---|
| Cetirizine hydrochloride | 2 g |
| Glycerin | 50 g |
| Urea | 20 g |
| Benzalkonium bromide | 0.2 g |
| Sodium chloride | 2 g |
| Distilled water | appropriate amount |

Adding distilled water to make up to 100 ml, and adjusting pH value to 5.0 with an appropriate amount of 10% sodium carbonate solution at the same time.

The preparation process was the same as that of Example 1. Finally, distilled water was added to make up to 100 ml and the resulted solution was uniformly mixed. Meanwhile, an appropriate amount of 10% sodium carbonate solution was added to adjust pH to 5.0.

Example 5

| | |
|---|---|
| Cetirizine hydrochloride | 0.5 g |
| Glycerin | 5 g |
| Urea | 1 g |
| Benzalkonium bromide | 0.02 g |
| Sodium chloride | 0.5 g |
| Distilled water | appropriate amount |

Adding distilled water to make up to 100 ml, and adjusting pH value to 4.5 with an appropriate amount of 10% sodium hydroxide solution at the same time.

The preparation process was the same as that of Example 1. Finally, distilled water was added to make up to 100 ml and the resulted solution was uniformly mixed. Meanwhile, an appropriate amount of 10% sodium hydroxide solution was added to adjust pH to 4.5.

Example 6

| | |
|---|---|
| cetirizine hydrochloride | 1.5 g |
| Glycerin | 40 g |
| Urea | 6 g |
| Benzalkonium bromide | 0.15 g |
| Sodium chloride | 1.5 g |
| Distilled water | appropriate amount |

Adding distilled water to make up to 100 ml, and adjusting pH value to 7.0 with an appropriate amount of 10% sodium carbonate solution at the same time.

The preparation process was the same as that of Example 1. Finally, distilled water was added to make up to 100 ml and the resulted solution was uniformly mixed. Meanwhile, an appropriate amount of 10% sodium carbonate solution was added to adjust pH to 7.0.

Example 7

| | |
|---|---|
| Cetirizine hydrochloride | 1 g |
| Glycerin | 10 g |
| Urea | 5 g |
| Benzalkonium bromide | 0.05 g |
| Sodium chloride | 1 g |
| Distilled water | appropriate amount |

Adding distilled water to make up to 100 ml, and adjusting pH value to 5.0 with an appropriate amount of 10% sodium bicarbonate solution at the same time.

The preparation process was the same as that of Example 1. Finally, distilled water was added to make up to 100 ml and the resulted solution was uniformly mixed. Meanwhile, an appropriate amount of 10% sodium bicarbonate solution was added to adjust pH to 5.0.

Example 8

| | |
|---|---|
| Cetirizine hydrochloride | 10 g |
| Glycerin | 100 g |
| Urea | 40 g |
| Benzalkonium bromide | 0.9 g |
| Sodium chloride | 8 g |
| Distilled water | appropriate amount |

Adding distilled water to make up to 1000 ml, and adjusting pH value to 5.5 with an appropriate amount of 10% sodium hydroxide solution at the same time.

The preparation process was the same as that of Example 1. Finally, distilled water was added to make up to 1000 ml and the resulted solution was uniformly mixed. Meanwhile, an appropriate amount of 10% sodium hydroxide solution was added to adjust pH to 5.5.

Example 9

| Cetirizine hydrochloride | 3 g |
| Glycerin | 200 g |
| Urea | 10 g |
| Benzalkonium bromide | 1 g |
| Sodium chloride | 9 g |
| Distilled water | appropriate amount |

Adding distilled water to make up to 1000 g, and adjusting pH value to 7.0 with an appropriate amount of 10% sodium hydroxide solution at the same time.

The preparation process was the same as that of Example 1. Finally, distilled water was added to make up to 1000 g and the resulted solution was uniformly mixed. Meanwhile, an appropriate amount of 10% sodium hydroxide solution was added to adjust pH to 7.0.

Example 10

| Cetirizine hydrochloride | 10 g |
| Glycerin | 350 g |
| Urea | 10 g |
| Benzalkonium bromide | 0.8 g |
| Sodium chloride | 5 g |
| Distilled water | appropriate amount |

Adding distilled water to make up to 1000 ml, and adjusting pH value to 6.5 with an appropriate amount of 10% sodium hydroxide solution at the same time.

The preparation process was the same as that of Example 1. Finally, distilled water was added to make up to 1000 ml and the resulted solution was uniformly mixed. Meanwhile, an appropriate amount of 10% sodium hydroxide solution was added to adjust pH to 6.5.

Example 11

| Cetirizine hydrochloride | 10 g |
| Glycerin | 400 g |
| Urea | 30 g |
| Benzalkonium bromide | 1.2 g |
| Sodium chloride | 15 g |
| Distilled water | appropriate amount |

Adding distilled water to make up to 1000 ml, and adjusting pH value to 6.0 with an appropriate amount of 10% sodium hydroxide solution at the same time.

The preparation process was the same as that of Example 1. Finally, distilled water was added to make up to 1000 ml and the resulted solution was uniformly mixed. Meanwhile, an appropriate amount of 10% sodium hydroxide solution was added to adjust pH to 6.0.

Part II: pharmaceutical solution of L-cetirizine hydrochloride and preparations thereof

Example 12

| | |
|---|---|
| L-cetirizine hydrochloride | 0.2 g |
| Distilled water | appropriate amount |
| Propanediol | 10 g |
| Sodium benzoate | 0.01 g |
| Urea | 1 g |
| Sodium chloride | 0.01 |

Adding distilled water to make up to 100 ml, and adjusting pH value to 7.0 with an appropriate amount of 10% sodium hydroxide solution at the same time.

Preparation process: 0.2 g L-cetirizine hydrochloride was dissolved in an appropriate amount of distilled water under ambient temperature and pressure; respective amounts of the components according to the above formula, i.e., 10 g propanediol, 1 g urea, 0.01 g sodium benzoate and 0.01 g sodium, were then added; distilled water was finally added to make up to 100 ml, uniformly stirred and mixed; an appropriate amount of 10% sodium hydroxide solution was added to adjust pH to 7.0 at the same time; and the resulted solution was packed, sealed and tested before obtaining the final product.

Example 13

| | |
|---|---|
| L-cetirizine hydrochloride | 2 g |
| Distilled water | appropriate amount |
| Glycerin | 40 g |
| Benzalkonium bromide | 0.2 g |
| Urea | 3 g |
| Sodium chloride | 5 g |

Adding distilled water to make up to 100 ml, and adjusting pH value to 5.0 with an appropriate amount of 10% sodium carbonate solution at the same time.

Preparation process: 2 g L-cetirizine hydrochloride was dissolved in an appropriate amount of distilled water under ambient temperature and pressure; respective amounts of the components according to the above formula, i.e., 40 g glycerin, 3 g urea, 0.2 g benzalkonium bromide and 5 g sodium chloride, were then added; distilled water was finally added to make up to 100 ml, uniformly mixed; an appropriate amount of 10% sodium carbonate solution was added to adjust pH to 5.0 at the same time; and the resulted solution was packed, sealed and tested before obtaining the final product.

Example 14

| | |
|---|---|
| L-cetirizine hydrochloride | 1 g |
| Distilled water | appropriate amount |
| Glycerin | 37.8 g |
| Urea | 2 g |
| Benzalkonium bromide | 0.1 g |
| Sodium chloride | 0.9 g |

Adding distilled water to make up to 100 ml, and adjusting pH value to 6.0 with an appropriate amount of 10% sodium hydroxide solution at the same time.

Preparation process: 1 g L-cetirizine hydrochloride was dissolved in an appropriate amount of distilled water under ambient temperature and pressure; respective amounts of the components according to the above formula, i.e., 37.8 g glycerin, 2 g urea, 0.1 g benzalkonium bromide and 0.9 g sodium chloride, were then added; distilled water was finally added to make up to 100 ml, uniformly mixed; an appropriate amount of 10% sodium hydroxide solution was added to adjust pH to 6.0 at the same time; and the resulted solution was packed, sealed and tested before obtaining the final product.

Example 15

| | |
|---|---|
| L-cetirizine hydrochloride | 1.2 g |
| distilled water | appropriate amount |
| Glycerin | 45.4 g |
| Urea | 2.4 g |
| benzalkonium bromide | 0.12 g |
| Sodium chloride | 1.08 g |

Adding distilled water to make up to 100 ml, and adjusting pH value to 6.0 with an appropriate amount of 10% sodium hydroxide solution at the same time.

The preparation process was the same as that of Example 3.

Example 16

| | |
|---|---|
| L-cetirizine hydrochloride | 1 g |
| Distilled water | appropriate amount |
| Glycerin | 37.8 g |
| Urea | 2 g |
| Benzalkonium bromide | 0.1 g |
| Sodium chloride | 0.9 g |
| Orange essence | 0.1 g |

Adding distilled water to make up to 100 ml, and adjusting pH value to 6.0 with an appropriate amount of 10% sodium hydroxide solution at the same time.

Preparation process: 1 g L-cetirizine hydrochloride was dissolved in an appropriate amount of distilled water under ambient temperature and pressure; respective amoutns of the components according to the above formula, i.e., 37.8 g glycerin, 2 g urea, 0.1 g benzalkonium bromide, 0.9 g sodium chloride and 0.1 g orange essence, were then added; distilled water was finally added to make up to 100 ml, uniformly mixed; an appropriate amount of 10% sodium hydroxide solution was added to adjust pH to 6.0 at the same time; and the resulted solution was packed, sealed and tested before obtaining the final product.

Example 17

| | |
|---|---|
| L-cetirizine hydrochloride | 1 g |
| Distilled water | appropriate amount |
| Glycerin | 37.8 g |
| Urea | 2 g |
| Benzalkonium bromide | 0.1 g |
| Sodium chloride | 0.9 g |
| Clove Oil | 0.02 g |

Adding distilled water to make up to 100 ml, and adjusting pH value to 6.0 with an appropriate amount of 10% sodium hydroxide solution at the same time.

Preparation process: 1 g L-cetirizine hydrochloride was dissolved in an appropriate amount of distilled water under ambient temperature and pressure; respective amounts of the components according to the above formula, i.e., 37.8 g glycerin, 2 g urea, 0.1 g benzalkonium bromide, 0.5 g sodium chloride and 0.02 g clove Oil, were then added; distilled water was finally added to make up to 100 ml, uniformly mixed; an appropriate amount of 10% sodium hydroxide solution was added to adjust pH to 6.0 at the same time; and the resulted solution was packed, sealed and tested before obtaining the final product.

Example 18

| L-cetirizine hydrochloride | 1 g |
| Distilled water | appropriate amount |
| Glycerin | 37.8 g |
| Urea | 2 g |
| Benzalkonium bromide | 0.1 g |
| Sodium chloride | 0.9 g |
| Apple essence | 1 g |

Adding distilled water to make up to 100 ml, and adjusting pH value to 6.0 with an appropriate amount of 10% sodium hydroxide solution at the same time.

Preparation process: 1 g L-cetirizine hydrochloride was dissolved in an appropriate amount of distilled water under ambient temperature and pressure; respective amounts of the components according to the above formula, i.e., 37.8 g glycerin, 2 g urea, 0.1 g benzalkonium bromide, 0.5 g sodium chloride and 1 g apple essence, were then added; distilled water was finally added to make up to 100 ml, uniformly mixed; an appropriate amount of 10% sodium hydroxide solution was added to adjust pH to 6.0 at the same time; and the resulted solution was packed, sealed and tested before obtaining the final product.

Example 19

| L-cetirizine hydrochloride | 1 g |
| Distilled water | appropriate amount |
| Propanediol | 10 g |
| Urea | 2 g |
| Benzalkonium bromide | 0.2 g |
| Sodium chloride | 1.5 g |
| Fragrant | 0.1 g |

Adding distilled water to make up to 100 ml, and adjusting pH value to 6.0 with an appropriate amount of 10% sodium hydroxide solution at the same time.

Preparation process: 1 g L-cetirizine hydrochloride was dissolved in an appropriate amount of distilled water under ambient temperature and pressure; respective amounts of the components according to the above formula, i.e., 10 g propanediol, 2 g urea, 0.2 g benzalkonium bromide, 1.5 g sodium chloride and 0.1 g flavoring agent, were then added; distilled water was finally added to make up to 100 ml, uniformly mixed, an appropriate amount of 10% sodium hydroxide solution was added to adjust pH to 6.0 at the same time; and the resulted solution was packed, sealed and tested before obtaining the final product.

Example 20

| L-cetirizine hydrochloride | 0.2 g |
| Distilled water | appropriate amount |
| Glycerin | 10 g |
| Urea | 1 g |
| Benzalkonium bromide | 0.01 g |
| Sodium chloride | 0.01 g |
| Banana essence | 0.05 g |

Adding distilled water to make up to 100 ml, and adjusting pH value to 5.0 with an appropriate amount of 10% sodium carbonate solution at the same time.

Preparation process: 0.2 g L-cetirizine hydrochloride was dissolved in an appropriate amount of distilled water under ambient temperature and pressure; respective amounts of the components according to the above formula, i.e., 10 g glycerin, 1 g urea, 0.01 g benzalkonium bromide, 0.01 g sodium chloride and 0.05 g banana essence, were then added; distilled water was finally added to make up to 100 ml, uniformly mixed; an appropriate amount of 10% sodium hydroxide solution was added to adjust pH to 6.0 at the same time; and the resulted solution was packed, sealed and tested before obtaining the final product.

Example 21

| L-cetirizine hydrochloride | 2 g |
| Distilled water | appropriate amount |
| Glycerin | 40 g |
| Urea | 3 g |
| Benzalkonium bromide | 0.2 g |
| Sodium chloride | 5 g |
| Banana essence | 1 g |

Adding distilled water to make up to 100 ml, and adjusting pH value to 5.0 with an appropriate amount of 10% sodium carbonate solution at the same time.

Preparation process: 2 g L-cetirizine hydrochloride was dissolved in an appropriate amount of distilled water under ambient temperature and pressure; respective amounts of the components according to the above formula, i.e., 40 g glycerin, 3 g urea, 0.2 g benzalkonium bromide, 5 g sodium chloride and 1 g banana essence, were then added; distilled water was finally added to make up to 100 ml, uniformly mixed; an appropriate amount of 10% sodium hydroxide solution was added to adjust pH to 6.0 at the same time; and the resulted solution was packed, sealed and tested before obtaining the final product.

Example 22

| L-cetirizine hydrochloride | 1 g |
| Distilled water | appropriate amount |
| Glycerin | 37.8 g |
| Urea | 2 g |
| Sodium benzoate | 0.1 g |
| Sodium chloride | 0.9 g |

Adding distilled water to make up to 100 ml, and adjusting pH value to 6.0 with an appropriate amount of 10% sodium hydroxide solution at the same time.

Preparation process: 1 g L-cetirizine hydrochloride was dissolved in an appropriate amount of distilled water under ambient temperature and pressure; respective amounts of the components according to the above formula, i.e.,

37.8g glycerin, 2 g urea, 0.1 g sodium benzoate and 0.9 g sodium chloride, were then added; distilled water was finally added to make up to 100 ml, uniformly mixed; an appropriate amount of 10% sodium hydroxide solution was added to adjust pH to 6.0 at the same time; and the resulted solution was packed, sealed and tested before obtaining the final product.

Example 23

| | |
|---|---|
| L-cetirizine hydrochloride | 1 g |
| Distilled water | appropriate amount |
| Glycerin | 37.8 g |
| Urea | 2 g |
| Chlorhexidine acetate | 0.05 g |

Adding distilled water to make up to 100 ml, and adjusting pH value to 6.0 with an appropriate amount of 10% sodium hydroxide solution at the same time.

Preparation process: 1 g L-cetirizine hydrochloride was dissolved in an appropriate amount of distilled water under ambient temperature and pressure; respective amounts of the components according to the above formula, i.e., 37.8 g glycerin, 2 g urea and 0.05 g chlorhexidine acetate, were then added; distilled water was finally added to make up to 100 ml, uniformly mixed; an appropriate amount of 10% sodium hydroxide solution was added to adjust pH to 6.0 at the same time; and the resulted solution was packed, sealed and tested before obtaining the final product.

Example 24 Liniment

| | |
|---|---|
| L-cetirizine hydrochloride | 0.2 g |
| Distilled water | appropriate amount |
| Glycerin | 10 g |
| Urea | 1 g |
| Benzalkonium bromide | 0.01 g |
| Sodium chloride | 0.01 g |
| Banana essence | 0.05 g |

Adding distilled water to make up to 100 ml, and adjusting pH value to 5.0 with an appropriate amount of 10% sodium carbonate solution at the same time.

The preparation process was the same as that of Example 20.

Example 25 Drops

| | |
|---|---|
| L-cetirizine hydrochloride | 2 g |
| Distilled water | appropriate amount |
| Glycerin | 40 g |
| Urea | 3 g |
| Benzalkonium bromide | 0.2 g |
| Sodium chloride | 5 g |
| Banana essence | 1 g |

Adding distilled water to make up to 100 ml, and adjusting pH value to 5.0 with an appropriate amount of 10% sodium carbonate solution at the same time.

The preparation process was the same as that of Example 21.

Example 26 Spray

| L-cetirizine hydrochloride | 1 g |
| Distilled water | appropriate amount |
| Glycerin | 37.8 g |
| Urea | 2 g |
| Sodium benzoate | 0.1 g |
| Sodium chloride | 0.9 g |

Adding distilled water to make up to 100 ml, and adjusting pH value to 6.0 with an appropriate amount of 10% sodium hydroxide solution at the same time.

The preparation process was the same as that of Example 22.

Example 27 Aerosol

| L-cetirizine hydrochloride | 1 g |
| Distilled water | appropriate amount |
| Glycerin | 37.8 g |
| Urea | 2 g |
| Chlorhexidine acetate | 0.05 g |
| Dichlorodifluoromethane | 20 g |

Adding distilled water to make up to 100 ml, and adjusting pH value to 6.0 with an appropriate amount of 10% sodium hydroxide solution at the same time.

The preparation process was the same as that of Example 23.

As the pharmaceutical solution of L-cetirizine hydrochloride according to the present invention and the preparation method thereof are described in reference to the preferred embodiments, the modifications and equivalent changes would be apparent for the person skilled in the art and thus are encompassed in the scope of the present application.

Part III: Pharmacodynamic studies of the pharmaceutical solution according to the present invention

Example 28: Anti-pruritic effects of the pharmaceutical solution of racemic cetirizine hydrochloride on 4-aminopyridine (4-AP) induced licking response in mice models

1. Experimental objective

To study the anti-pruritic effects of the pharmaceutical solution of racemic cetirizine hydrochloride on 4-aminopyridine (4-AP) induced licking response in mice models.

2. Tested medicines

2.1 Ordinary liniment of cetirizine hydrochloride, prepared according to Example 5 in CN1634063A, wherein the concentration of cetirizine hydrochloride is 10 mg/ml;

2.2 Liposome liniment of cetirizine hydrochloride, prepared according to Example 3 in CN1634063A, wherein the concentration of cetirizine hydrochloride is 12 mg/ml;

2.3 Aerosol of cetirizine hydrochloride, prepared according to Example 9 in CN1634063A, wherein the concentration of cetirizine hydrochloride is 10 mg/ml;

2.4 pharmaceutical solution I of racemic cetirizine hydrochloride, prepared according to Example 3 of the present invention, wherein the concentration of cetirizine hydrochloride is 10 mg/ml.

3. Grouping and administration

3.1 Normal control group, topical administration of physiological saline;
3.2 Model control group, topical administration of physiological saline;
3.3 Cetirizine aerosol group, topical administration of the aerosol of cetirizine hydrochloride;
3.4 Cetirizine liposome liniment group, topical administration of the liposome liniment of cetirizine hydrochloride;
3.5 Cetirizine ordinary liniment group, topical administration of the ordinary liniment of cetirizine hydrochloride;
3.6 Cetirizine solution group, topical administration of the pharmaceutical solution I of racemic cetirizine hydrochloride.

Note: Corresponding medicines or physiological saline are administrated in an equal volume (0.025 ml) for each group.

4. Experimental method

60 mice (body weight of 20-25 g) were randomly divided into six groups (10 mice each group, including 5 female and 5 male), i.e. normal control group, model control group, cetirizine aerosol group, cetirizine liposome liniment group, cetirizine ordinary liniment group and cetirizine solution group. All mice were denuded in the area of neck and back with 8% sodium sulfide, where the denuded area is 1.5 cm x 2 cm. For each group, corresponding topical medicine was applied or sprinkled on the specific denuded area of the mice (1.5 cm x 2 cm) in an equal volume (0.025 ml), except for the normal control group and the model control group in which physiological saline was applied in an equal volume (0.025 ml). After 30 min, all mice except for those in the normal control group were subcutaneously injected with 0.01% 4-aminopyridine (4-AP) at a dose of 1 mg/kg. After administration, all mice were placed in a cage. After about 30 seconds, the mice showed the behavior of repeatedly turning around to lick the back side, i.e. licking response. A continuous licking before a short pause was defined as one licking, and the number of licking within 30 min after the administration of 4-AP was recorded. The number of licking was used as an indicator for assessing the anti-pruritic effects. Difference between groups was compared.

5. Experimental results

The results of the experiments showed that, compared to the model group, there were significant differences in the anti-pruritic effects of cetirizine in each administration group on the 4-aminopyridine (4-AP)-induced licking response in mice models. Compared to the cetirizine aerosol group, the cetirizine liposome liniment group and the cetirizine ordinary liniment group, the antipruritic effects of cetirizine solution group on the 4-aminopyridine (4-AP)-induced licking response in mice models showed significant difference (Table 1).

Table 1 Comparison of anti-pruritic effects of the pharmaceutical solution of racemic cetirizine hydrochloride and other topical preparations on the 4-aminopyridine (4-AP)-induced licking response in mice models

| Groups | Number of mice | Number of licking ($\bar{x}\pm s$) |
| --- | --- | --- |
| Normal control group | 10 | $6.0\pm1.8$ |
| Model control group | 10 | $93.5\pm21.2$ |
| Cetirizine ointment group | 10 | $80.2\pm16.5$* |
| Cetirizine liposome liniments group | 10 | $83.4\pm17.9$* |
| Cetirizine ordinary liniments group | 10 | $84.0\pm14.2$* |
| Cetirizine solution group | 10 | $61.3\pm18.1$**▲★■ |

*Compared to the model control group, p<0.05;
** Compared to the model control group, p<0.01;
★Compared to the cetirizine aerosol group, p<0.05;
▲Compared to the cetirizine liposome liniment group, p<0.05;
■Compared to the cetirizine ordinary liniment group, p<0.05.

Example 29: Antipruritic effects of the pharmaceutical solution of L-cetirizine hydrochloride on 4-aminopyridine (4-AP) induced licking response in mice models

1. Experimental objective

To study the antipruritic effects of the pharmaceutical solution of L-cetirizine hydrochloride on 4-aminopyridine (4-AP) induced licking response in mice models.

2. Tested medicines

2.1 Aerosol of L-cetirizine hydrochloride, prepared according to Example 7 in CN1957942A, wherein the concentration of L-cetirizine hydrochloride is 10 mg/ml;
2.2 Liniment of L-cetirizine hydrochloride, prepared according to Example 1 in CN1957942A, wherein the concentration of cetirizine hydrochloride is 10 mg/ml;
2.3 Solution of L-cetirizine hydrochloride, prepared according to Example 14 of the present invention, wherein the concentration of cetirizine hydrochloride is 10 mg/ml.

3. Grouping and administration

3.1 Normal control group, topical administration of physiological saline;
3.2 Model control group, topical administration of physiological saline;
3.3 L-Cetirizine aerosol group, topical administration of the aerosol of L-cetirizine hydrochloride;
3.4 L-Cetirizine liniment group, topical administration of the liniment of L-cetirizine hydrochloride;
3.5 L-Cetirizine solution group, topical administration of the solution of L-cetirizine hydrochloride.

Note: Corresponding medicines or physiological saline were administrated in an equal volume (0.025 ml) for each group.

4. Experimental method

50 mice (body weight of 20-25 g) were randomly divided into 5 groups with 10 mice in each group including 5 female and 5 male. All mice were denuded in the area of neck and back with 8% sodium sulfide, wherein the denuded area is 1.5 cm x 2 cm. For each group, corresponding topical medicine was applied or sprinkled on the specific denuded area of the mice (1.5 cm x 2 cm) in an equal volume (0.025 ml), except for the normal control group and the model control group in which physiological saline was applied in an equal volume (0.025 ml). After 30 min, all mice except for those in the normal control group were subcutaneously injected with 0.01% 4-aminopyridine (4-AP) at a dose of 1 mg/kg. After administration, all mice were placed in a cage. After about 30 seconds, the mice showed the behavior of repeatedly turning around to lick the back side, i.e. licking response. A continuous licking before a short pause was defined as one licking, and the number of licking within 30 min after the administration of 4-AP was recorded. The number of licking was used as an indicator for assessing the anti-pruritic effects. The less the number of the licking was, the better the anti-pruritic effects were. Difference between groups was compared.

5. Experimental results

1) Compared to the model group, there were significant differences in the antipruritic effects of L-cetirizine hydrochloride in the L-Cetirizine liniment group, the L-Cetirizine aerosol group and the L-Cetirizine hydrochloride solution group on the 4-aminopyridine (4-AP)-induced mice response in mice models. (Table 2)
2) Compared to the L-Cetirizine aerosol group and the L-Cetirizine liniment group, the antipruritic effects of the L-Cetirizine solution group on the 4-aminopyridine (4-AP)-induced licking response in mice models showed significant difference. (Table 2)

**EP 2 465 503 A1**

Table 2: Antipruritic effects of the aerosol of L-cetirizine hydrochloride on the 4-aminopyridine (4-AP)-induced licking response in mice models

| Groups | Number of mice | Number of licking ($\bar{x}\pm$s) |
|---|---|---|
| Normal control group | 10 | $10.0\pm5.2$ |
| Model control group | 10 | $90.5\pm19.2$ |
| L-cetirizine liniment group | 10 | $81.2\pm17.5$* |
| L-cetirizine aerosol group | 10 | $79.6\pm15.0$* |
| L-cetirizine solution group | 10 | $55.3\pm14.2$****★■ |

*Compared to the model control group, $p<0.05$;
** Compared to the model control group, $p<0.01$;
★Compared to the L-cetirizine aerosol group, $p<0.05$;
■Compared to the L-cetirizine liniment group, $p<0.05$.

Example 30: Effects of the pharmaceutical solution of racemic cetirizine hydrochloride on IgE induced skin immediate-phase (IP) allergic reaction model

1. Experimental objective

To study the effects of the pharmaceutical solution of racemic cetirizine hydrochloride on IgE induced skin immediate-phase (IP) allergic reaction model.

2. Tested medicines

2.1 Ointment of cetirizine hydrochloride, prepared according to Example 8 in CN1634063A, wherein the concentration of cetirizine hydrochloride is 0.3%;
2.2 pharmaceutical solution II of racemic cetirizine hydrochloride, prepared according to Example 9 of the present invention, wherein the concentration of cetirizine hydrochloride is 0.3%.

3. Grouping and administration

3.1 Normal control group, topical administration of physiological saline;
3.2 Model control group, topical administration of physiological saline;
3.3 Cetirizine ointment group, topical administration of the ointment of cetirizine hydrochloride;
3.4 Cetirizine solution group, topical administration of the pharmaceutical solution II of racemic cetirizine hydrochloride.

Note: the administration dose (or physiological saline) is 0.05 g for each group.

4. Experimental method

40 mice (body weight of 20-25 g) were randomly divided into 4 groups, i.e. normal control group, model control group, cetirizine ointment group and cetirizine solution group, with 10 mice in each group including 5 female and 5 male. The mice in the normal control group were denuded in the area of ears (1 cm x 1 cm). The mice in other groups were injected with 0.5 ml of anti-DNP IgE monoclonal antibody by tail intravenous injection, and excited by applying 50 $\mu$l of 0.15% DNFB solution on both ears after 24 h. One hour before the excitation, the thickness of the mice's ears were determined by a thickness detector (Harbin Measuring & Cutting Tool Group CO., LTD., precision: 1 $\mu$m) and recorded, and then the ointment of cetirizine hydrochloride and the pharmaceutical solution II of racemic cetirizine hydrochloride, each in an amount of 0.05 g, were applied on the denuded area of the mice's ears (1 cm x 1 cm). Physiological saline was applied to the normal control group and the model control group in an equal amount (0.05 g). One hour after the excitation, the thickness ($\mu$m) of the mice's ears were determined by a thickness detector (Harbin Measuring & Cutting Tool Group CO., LTD., precision: 1 µm). The difference of the thicknesses of the mice's ears one hour after the DNFB excitation and one hour before the DNFB excitation was defined as the swelling degree (µm) of the mice's ears. The swelling degree (µm) of the mice's ears was used as an indicator for assessing the effects of cetirizine hydrochloride on IgE induced skin immediate-phase (IP) allergic reaction. T test was performed for the swelling degree, and the difference between groups was compared.

5. Experimental results

The results showed that, compared to the model group, the cetirizine ointment group and the cetirizine solution group showed significant effects on the swelling degree of IgE induced skin immediate-phase (IP) allergic reaction model. Compared to the cetirizine ointment group, the effect of the cetirizine solution group on the swelling degree of IgE induced skin immediate-phase (IP) allergic reaction model showed significant difference, indicating that cetirizine hydrochloride liniment II has better anti-allergic effects. (Table 3)

Table 3 Comparison of the effects between the pharmaceutical solution of racemic cetirizine hydrochloride and the ointment of cetirizine hydrochloride on the ear swelling degree of IgE induced allergic skin immediate-phase (IP) allergic reaction model

| Groups | Number of mice | Ear swelling degree (µm) one hour after DNFB excitation |
|---|---|---|
| Normal control group | 10 | $0.6 \pm 0.4$ |
| Model control group | 10 | $12.8 \pm 3.2$ |
| Cetirizine ointment group | 10 | $7.8 \pm 2.4$** |
| Cetirizine solution group | 10 | $4.3 \pm 2.2$**■ |

** Compared to the model control group, $p < 0.01$;
■Compared to the cetirizine ointment group, $p < 0.05$.

Example 31: Anti-inflammatory effects of the pharmaceutical solution of L-cetirizine hydrochloride on IgE induced skin immediate-phase (IP) allergic reaction model

1. Experimental objective

To study the effects of the pharmaceutical solution of L-cetirizine hydrochloride on IgE induced skin immediate-phase (IP) allergic reaction model.

2. Tested medicines

2.1 Aerosol of L-cetirizine hydrochloride, prepared according to Example 7 in CN1957942A, wherein the concentration of L-cetirizine hydrochloride is 10 mg/ml;
2.2 Liniment of L-cetirizine hydrochloride, prepared according to Example 1 in CN1957942A, wherein the concentration of L-cetirizine hydrochloride is 10 mg/ml;
2.3 Solution of L-cetirizine hydrochloride, prepared according to Example 14 in the present invention, wherein the concentration of L-cetirizine hydrochloride is 10 mg/ml.

3. Grouping and administration

3.1 Normal control group, topical administration of physiological saline;
3.2 Model control group, topical administration of physiological saline;
3.3 L-Cetirizine aerosol group, topical administration of aerosol of L-cetirizine hydrochloride;
3.4 L-Cetirizine liniment group, topical administration of liniment of L-cetirizine hydrochloride;
3.5 L-Cetirizine solution group, topical administration of the pharmaceutical solution of L-cetirizine hydrochloride.

Note: corresponding medicines or physiological saline were administered in an equal volume (0.025 ml) for each group.

4. Experimental method

50 mice (body weight of 20-25 g) were randomly divided into five groups with 10 mice in each group including 5 female and 5 male. The mice in the normal control group were denuded in the area of ears (1 cm x 1 cm). The mice in other groups were injected with 0.5 ml of anti-DNP IgE monoclonal antibody by tail intravenous injection, and excited by applying 50 $\mu$l of 0.15% DNFB solution on ears after 24 h. One hour before the excitation, the thickness of the mice's ears were determined by a thickness detector (Harbin Measuring & Cutting Tool Group CO., LTD., precision: 1 $\mu$m) and recorded, and then the liniment of L-cetirizine hydrochloride, the aerosol of L-cetirizine hydrochloride and the pharmaceutical solution of L-cetirizine hydrochloride were applied on the denuded area of the mice's ears (1 cm x 1 cm) in an equal amount (0.025 ml). The same amount of physiological saline was applied on the denuded area of the mice's ears for the normal control group and the model control group. One hour after the excitation, the thickness ($\mu$m) of the mice's ears were determined by a thickness detector (Harbin Measuring & Cutting Tool Group CO., LTD., precision: 1 $\mu$m). The difference of the thicknesses of the mice's ears one hour after the DNFB excitation and one hour before the DNFB excitation was defined as the swelling degree ($\mu$m) of the mice's ears. The swelling degree ($\mu$m) of the mice's ears was used as an indicator for assessing the effects of L-cetirizine hydrochloride on IgE induced allergic skin immediate-phase (IP) allergic reaction. The less the number was, the better the anti-inflammatory effects were. Difference between groups was compared. T test was performed for the swelling degree, and the difference between groups was compared.

5. Experimental results

1) Compared to the model group, the L-Cetirizine liniment group, the L-Cetirizine aerosol group and the L-Cetirizine hydrochloride solution group showed significant effects on the swelling degree in the IgE induced allergic skin immediate-phase (IP) allergic reaction model. (Table 4)
2) Compared to the L-Cetirizine aerosol group and the L-Cetirizine liniment group, the effects of the L-Cetirizine solution group on the swelling degree in the IgE induced skin immediate-phase (IP) allergic reaction model showed significant difference, indicating that the anti-inflammatory effects of the pharmaceutical solution of L-Cetirizine hydrochloride were more significant. (Table 4)

Table 4: Effects of the pharmaceutical solution of L-Cetirizine hydrochloride on the ear swelling degree in the IgE induced skin immediate-phase (IP) allergic reaction model

| Group | Number of mice | Ear swelling degree ($\mu$m) one hour after DNFB excitation |
|---|---|---|
| Normal control group | 10 | 0.6±0.4 |
| Model control group | 10 | 12.8±3.2 |
| L-Cetirizine liniment group | 10 | 8.0±2.6* |
| L-Cetirizine aerosol group | 10 | 7.8±2.4* |
| L-Cetirizine solution group | 10 | 4.3±2.2**■★ |

*Compared to the model control group, p<0.05;
** Compared to the model control group, p<0.01;
■Compared to the L-cetirizine aerosol group, p<0.05;
★Compared to the L-cetirizine liniment group, p<0.05.

Example 32: Effects of the pharmaceutical solution of racemic cetirizine hydrochloride on IgE induced allergic skin late phase (LP) model

1. Experimental objective

To study the effects of the pharmaceutical solution of racemic cetirizine hydrochloride on IgE induced allergic skin late phase (LP) model.

2. Tested medicines

2.1 Liniment of cetirizine hydrochloride, prepared according to Example 5 in CN1634063A, wherein the concentration of cetirizine hydrochloride is 10 mg/ml;

2.2 pharmaceutical solution of racemic cetirizine hydrochloride, prepared according to Example 7 of the present invention, wherein the concentration of cetirizine hydrochloride is 10 mg/ml.

3. Grouping and administration

3.1 Normal control group, topical administration of physiological saline;

3.2 Model control group, topical administration of physiological saline;

3.3 Liniment group, topical administration of liniment of racemic cetirizine hydrochloride;

3.4 Solution group, topical administration of the pharmaceutical solution of racemic cetirizine hydrochloride.

Note: the administration dose is 0.025 ml for each group.

4. Experimental method

40 mice (body weight of 20-25 g) were randomly divided into four groups, i.e., normal control group, model control group, liniment group, and solution group, with 10 mice in each group including 5 female and 5 male. The mice in the normal control group were denuded in the area of ears (1 cm x 1 cm). The mice in other groups were injected with 0.5 ml of anti-DNP IgE monoclonal antibody by tail intravenous injection, and excited by applying 50 $\mu$l of 0.15% DNFB solution on ears after 24 h. One hour before the excitation and 12 hours after the excitation, the liniment of racemic cetirizine hydrochloride and the pharmaceutical solution of racemic cetirizine hydrochloride were applied respectively to the denuded area of the mice's ears (1 cm x 1 cm) in an equal amount (0.025 ml) for twcie. The same amount of physiological saline was applied on the denuded area of the mice's ears for the normal control group and the model control group. The thickness ($\mu$m) of the mice's ears were determined by a thickness detector (Harbin Measuring & Cutting Tool Group CO., LTD., precision: 1 $\mu$m) one hour before the excitation, and 1 hour, 12 hours and 24 hours after the excitation. The difference of the thicknesses of the mice's ears one hour after the DNFB excitation and one hour before the DNFB excitation was defined as the swelling degree ($\mu$m) of the mice's ears. The swelling degree ($\mu$m) of the mice's ears was used as an indicator for assessing the effects of the solution of racemic cetirizine hydrochloride on IgE induced skin late-phase (LP) allergic reaction. T test was performed for the swelling degree, and the difference between groups was compared.

5. Experimental results

The results showed that, compared to the model group, the effects of the liniment group and the solution group on the swelling degree of IgE induced skin late-phase (LP) allergic reaction model showed significant differences. Compared to the liniment group, the effect of the solution group on the swelling degree of IgE induced skin late-phase (LP) allergic reaction model showed significant difference, indicating that the pharmaceutical solution of racemic cetirizine hydrochloride has more significant anti-allergic effects. (Table 5)

Table 5: Effects of the pharmaceutical solution of racemic cetirizine hydrochloride on the ear swelling degree in the IqE induced skin late-phase (LP) allergic reaction model

| Groups | Numbers of mice | Ear swelling degree ($\mu$m) 24 hours after DNFB excitation |
|---|---|---|
| Normal control group | 10 | 0.5$\pm$0.4 |
| Model control group | 10 | 23.2$\pm$3.4 |
| Liniment group | 10 | 16.0$\pm$2.4** |

(continued)

| Groups | Numbers of mice | Ear swelling degree ($\mu$m) 24 hours after DNFB excitation |
|---|---|---|
| Solution group | 10 | $7.4\pm2.8$**■ |

**Compared to the model control group, $p<0.01$;
■Compared to the liniment group, $p<0.05$.

Example 33: Therapeutic observations of the pharmaceutical solution of racemic cetirizine hydrochloride on skin swelling and itching caused by mosquito stings

1. Clinical symptoms

Upon piercing into the skin, the mosquitoes secrete formic acid through their mouthpart, resulting in the feel of stinging. There may be erythema, papules or wheal in the stung site with a dark-red blood spot in the center of the injured sites, which can not completely fade when press-diagnosed. It is characterized by a pale circle around the pain points. The degrees of redness and itching varies, in which some people have no symptoms after being stung, and some ones only feel mild itching and slight pain. Some ones with allergy, however, may show a significant swelling, and even large areas of stasis spot, accompanied by intense itching and burning feeling.

2. Clinical information

200 patients aging 2-70 years old and stung by mosquitoes were recruited with 120 males and 80 females. Besides skin swelling and itching caused by mosquito stings, the patients were physically healthy and were not subjected to the administration of other medicines within 7 days before the treatment of the present invention.

3. Therapy strategies

3.1 Medicines

pharmaceutical solution I of racemic cetirizine hydrochloride, prepared according to Example 3 of the present invention, wherein the concentration of cetirizine hydrochloride is 10 mg/ml;
pharmaceutical solution IV of racemic cetirizine hydrochloride, prepared according to Example 2 of the present invention, wherein the concentration of cetirizine hydrochloride is 5 mg/ml;
Tablets of cetirizine hydrochloride (specification: 10 mg/tablet, provided by Lunan Pharmaceutical Group Corporation);
Essential balm (specification: 5 mg/bottle, provided by Fujian Pacific Pharmaceutical CO., LTD.).

3.2 Administration and doses

High dose solution group: 50 patients. The pharmaceutical solution I of racemic cetirizine hydrochloride was applied on the affected site twice per day (morning and night);
Low dose solution group: 50 patients. The pharmaceutical solution IV of racemic cetirizine hydrochloride was applied on the affected site twice per day (morning and night);
Essential balm group: 50 patients. The essential balm was applied to the affected site twice per day (morning and night);
Cetirizine hydrochloride tablet group: 50 patients. Cetirizine hydrochloride tablets (10 mg/tablet) were given to the patients. A half tablet was orally administrated to children once per day, and one tablet was orally administrated to adult once per day. See the detailed specification of the cetirizine hydrochloride tablets.

3.3 Emergency treatment of adverse reactions

If exacerbation or other skin damage occurs after administration of the product, the administration should be stopped immediately and further medical assistance shall be given.

4. Efficacy analysis

4.1 Efficacy standard

4.1.1 Recovery: clinical symptoms disappeared, no swelling, no itching, and no recurrence within 72h follow-up;
4.1.2 Improvement- significant improvement in clinical symptoms, swelling reduction of at least 1/3, significantly reduced itching, no recurrence within 18h
4.1.3 Inefficacy: no significant improvement of clinical symptoms after two days' administration

4.2 Data recording and processing

For the high dose solution group, the low dose solution group, the essential balm group and the cetirizine hydrochloride tablet group, the numbers of patients with recovery, improvement (excluding the number of recovered patients), and inefficacy, and the average onset time (min) were recorded, respectively, in which the average onset time was the time from administration to significant reduction of the itching for each group.
The data processing was as follows:

$$\text{Recovery ratio} = \text{the number of patients with recovery} / \text{total number of patients in each group} \times 100\%;$$

$$\text{Improvement ratio} = \text{the number of patients with improvement} / \text{total number of patients in each group} \times 100\%;$$

$$\text{Inefficacy ratio} = \text{the number of patients with inefficacy} / \text{total number of patients in each group} \times 100\%;$$

$$\text{Efficacy ratio} = \text{Recovery ratio} + \text{Improvement ratio}$$

$x^2$ test of the efficacy ratio for each group was performed with Statistical software SPSS 11.0. If $x^2 > x^2_{0.05,1} = 3.84$, there was statistical difference provided that $p < 0.05$. The difference between each group was compared.

4.3 Results of treatment

Clinical results showed that, the pharmaceutical solution of cetirizine hydrochloride of the present invention had an efficiency of at least 90% on the skin swelling and itching caused by mosquito stings. The average onset time was 6 min and 4 min, respectively, for the high dose solution group (10 mg/ml) and the low dose solution group (5 mg/ml). There were no exacerbations and other adverse reactions after the solution of racemic cetirizine hydrochloride of the present invention was applied to the patients. The essential balm group showed an efficiency of 86% and an average onset time of 15 min for the skin swelling and itching caused by mosquito stings, and there were 6 cases of adverse reactions. This further indicates that the solution of racemic cetirizine hydrochloride has a less irritation and a better efficiency than the essential balm. For the therapeutic efficiency, compared to the cetirizine hydrochloride tablet group, there was significant difference for the high dose solution group and low dose solution group of racemic cetirizine hydrochloride on the skin swelling and itching caused by mosquito stings ($p < 0.05$). This indicates that the solution of racemic cetirizine hydrochloride had better therapeutic efficiency than the tablet of racemic cetirizine hydrochloride for the treatment of the skin swelling and itching caused by mosquito stings. The results were shown in table 6.

Table 6: Therapeutic efficiency of the pharmaceutical solution of racemic cetirizine hydrochloride and other treatment on the skin local swelling and itching caused by mosquito stings

| Treatment groups | No. of cases | Average onset time/min | Recovery ratio/% | Efficacy ratio/% | Inefficacy ratio/% | Cases with adverse reactions |
|---|---|---|---|---|---|---|
| Essential balm group | 50 | 15 | 78 | 86 | 14 | 6 |
| Cetirizine hydrochloride tablet group | 50 | - | 74 | 68 | 32 | 2 |
| Low dose solution group | 50 | 6 | 80 | 90▲ | 10 | 0 |
| High dose solution group | 50 | 4 | 82 | 94*▲ | 6 | 0 |

*Compared to the cetirizine hydrochloride tablet group, $p < 0.05$;
▲Compared to the essential balm group, $p < 0.05$.

Example 34: Therapeutic observations of the pharmaceutical solution of L-cetirizine hydrochloride on skin swelling and itching caused by mosquito stings

1. Clinical symptoms

Upon piercing into the skin, the mosquitoes secrete formic acid through their mouthpart, resulting in the feel of stinging. There may be erythema, papules or wheal in the stung site with a dark-red blood spot in the center of the injured sites, which can not completely fade when press-diagnosed. It is characterized by a pale circle around the pain points. The degrees of redness and itching varies, in which some people have no symptoms after being stung, and some ones only feel mild itching and slight pain. Some ones with allergy, however, may show a significant swelling, and even large areas of stasis spot, accompanied by intense itching and burning feeling.

2. Clinical information

300 aging 2-70 years old and stung by mosquitoes were recruited, with 180 males and 120 females. Besides skin swelling and itching caused by mosquito stings, the patients were physically healthy and were not subjected to the administration of other medicines within 7 days before the treatment of the present invention.

3. Therapy strategies

3.1 Medicines

3.1.1 Aerosol of L-cetirizine hydrochloride, prepared according to Example 7 in CN1957942A, wherein the concentration of L-cetirizine hydrochloride is 10 mg/ml;
3.1.2 Liniment of L-cetirizine hydrochloride, prepared according to Example 1 in CN1957942A, wherein the concentration of L-cetirizine hydrochloride is 10 mg/ml;
3.1.3 pharmaceutical solution A of L-cetirizine hydrochloride, prepared according to Example 14 of the present invention, wherein the concentration of L-cetirizine hydrochloride is 10 mg/ml;
3.1.4 pharmaceutical solution B of L-cetirizine hydrochloride, prepared according to Example 15 of the present invention, wherein the concentration of L-cetirizine hydrochloride is 12 mg/ml;
3.1.5 Essential balm (specification: 5 mg/bottle, provided by Fujian Pacific Pharmaceutical CO., LTD.).

3.2 Administration and doses

Low dose solution group: 60 patients. The solution A of L-cetirizine hydrochloride was applied on the affected site twice per day (morning and night);
High dose solution group: 60 patients. The solution B of L-cetirizine hydrochloride was applied on the affected site twice per day (morning and night);
Liniment group: 60 patients. The liniment of L-cetirizine hydrochloride was applied on the affected site twice per day (morning and night);
Aerosol group: 60 patients. The aerosol of L-cetirizine hydrochloride was applied on the affected site twice per day (morning and night);
Essential balm group: 60 patients. The essential balm was applied on the affected site twice per day (morningday and night).

3.3 Emergency treatment of adverse reactions

If exacerbation or other skin damage occurs after administration of the product, the administration should be stopped immediately and further medical assistance shall be given.

4. Efficacy analysis

4.1 Efficacy standard

4.1.1 Recovery: clinical symptoms disappeared, no swelling, no itching, and no recurrence within 72h follow-up;
4.1.2 Improvement: significant improvement in clinical symptoms, swelling reduction of at least 1/3, significantly reduced itching, no recurrence within 18h
4.1.3 Inefficacy: no significant improvement of clinical symptoms after two days' administration

4.2 Data recording and processing

For each treatment group, the numbers of patients with recovery, improvement (excluding the number of recovered patients), and inefficacy, and the average onset time (min) were recorded, respectively, in which the average onset time was the time from administration to significant reduction of the itching for each treatment group.
The data processing was as follows:

Recovery ratio = the number of patients with recovery / total number of patients in each group × 100%;

Improvement ratio = the number of patients with improvement / total number of patients in each group × 100%;

Inefficient ratio = the number of patients with inefficacy / total number of patients in each group × 100%;

Efficacy ratio = Recovery ratio + Improvement ratio

$x^2$ test of the efficacy ratio for each group was performed with Statistical software SPSS 11.0. If $x^2 > x^2_{0.05,1} = 3.84$, there was statistical difference provided that $p < 0.05$. The difference between each group was compared.

5. Clinical results

Clinical results showed that:

(1) The solution of L-cetirizine hydrochloride of the present invention had an efficiency of at least 90% on the skin swelling and itching caused by mosquito stings. The average onset time was 8 min and 9 min, respectively, for the high dose solution group (12 mg/ml) and the low dose solution group (10 mg/ml).
(2) The high dose solution group and low dose solution group of L-cetirizine hydrochloride showed significantly difference as compared to the essential balm group, the aerosol group and the liniment group regarding the therapeutic efficiency ($p < 0.05$), the average onset time ($p < 0.05$), and adverse reactions ($p < 0.05$).

This further indicates that the pharmaceutical solution of L-cetirizine hydrochloride has high therapeutic efficiency, low irritation on the skin and short anti-itching time for the treatment of the skin swelling and itching caused by mosquito stings. The results were shown in table 7.

Table 7: Therapeutic efficiency of the pharmaceutical solution of L-cetirizine hydrochloride on the skin local swelling and itching caused by mosquito stings

| Treatment groups | No. of cases | Recovery ratio/% | Efficacy ratio/% | Inefficacy ratio/% | Average onset time/min |
|---|---|---|---|---|---|
| Essential balm group | 60 | 65 | 70 | 20 | 15.5±5.5 |
| Aerosol group | 60 | 75 | 80 | 10 | 18.0±6.0 |
| Liniment group | 60 | 70 | 76.7 | 10 | 20.0±7.0 |
| Low dose solution group | 60 | 80 | 90■▲★ | 0■▲▲★ | 9.0±4.0■▲★ |
| High dose solution group | 60 | 85 | 93.3■▲★ | 0■▲▲★ | 8.0±3.0■▲★ |

■Compared to the liniment group, $p < 0.05$;
▲Compared to the essential balm group, $p < 0.05$;
★Compared to the aerosol group, $p < 0.05$.

**Claims**

1. A pharmaceutical solution of cetirizine hydrochloride, **characterized by** comprising the following components:

| | |
|---|---|
| Cetirizine hydrochloride | 0.1~2 parts by weight; |
| Alcohol having 3 or less carbon atoms | 1-50 parts by weight; |
| Preservative | 0.01~0.2 parts by weight; |
| Urea | 0.02~20 parts by weight; and |
| Inorganic salt | 0~5 parts by weight, |

Wherein said cetirizine hydrochloride is racemic cetirizine hydrochloride or L-cetirizine hydrochloride.

2. The pharmaceutical solution according to claim 1, **characterized by** comprising the following components:

| | |
|---|---|
| Cetirizine hydrochloride | 0.2~2 parts by weight; |
| Alcohol having 3 or less carbon atoms | 10-40 parts by weight; |
| Preservative | 0.01~0.2 parts by weight; |
| Urea | 1~5 parts by weight; and |
| Inorganic salt | 0~5 parts by weight, |

Wherein said cetirizine hydrochloride is L-cetirizine hydrochloride.

3. The pharmaceutical solution according to claim 2, **characterized by** comprising the following components:

| | |
|---|---|
| L-cetirizine hydrochloride | 0.5~1.5 parts by weight; |
| Alcohol having 3 or less carbon atoms | 15-40 parts by weight; |
| Preservative | 0.05~0.2 parts by weight; |
| Urea | 1~3 parts by weight; and |
| Inorganic salt | 0~5 parts by weight. |

4. The pharmaceutical solution according to claim 3, **characterized by** comprising the following components:

| | |
|---|---|
| L-cetirizine hydrochloride | 1 part by weight; |
| Alcohol having 3 or less carbon atoms | 15-40 parts by weight; |
| Preservative | 0.1 parts by weight; |
| Urea | 2 parts by weight; and |
| Inorganic salt | 0.5~1.5 parts by weight. |

5. The pharmaceutical solution according to any one of claims 2-4, **characterized in that** said alcohol having 3 or less carbon atoms is propanediol, glycerin, propanol, ethanol or any combination thereof; said preservative is one or two selected from benzalkonium bromide, chlorhexidine acetate, sodium benzoate and sorbic acid; and said inorganic salt is one or more selected from sodium chloride, potassium chloride, sodium sulfate, potassium sulfate, calcium chloride and sodium bicarbonate.

6. The pharmaceutical solution according to claim 5, **characterized in that** said alcohol having 3 or less carbon atoms is glycerin or 1,2-propanediol; said preservative is benzalkonium bromide or chlorhexidine acetate; and said inorganic salt is sodium chloride.

7. The pharmaceutical solution according to claim 6, **characterized in that** said alcohol having 3 or less carbon atoms is glycerin and said preservative is benzalkonium bromide.

8. The pharmaceutical solution according to any one of claims 2-4, **characterized in that** the pH of the pharmaceutical solution is 5.0~7.0.

9. The pharmaceutical solution according to claim 8, **characterized in that** the pH of the pharmaceutical solution is 6.0.

10. The pharmaceutical solution according to any one of claims 2-4, **characterized in that** said pharmaceutical solution is liniment, drops, aerosol or spray.

11. The pharmaceutical solution according to claim 5, **characterized in that** said pharmaceutical solution further comprises a flavoring agent which is orange peel oil, anise oil, citron oil, citron tincture, a fragrant, cinnamon water, cinnamon oil, banana essence, orange essence, lemon essence or apple essence.

12. The pharmaceutical solution according to claim 1, **characterized by** comprising the following components:

| | |
|---|---|
| Cetirizine hydrochloride | 0.1~2 parts by weight; |
| Alcohol having 3 or less carbon atoms | 1-50 parts by weight; |
| Preservative | 0.02~0.2 parts by weight; |
| Urea | 0.02~20 parts by weight; and |
| Inorganic salt | 0.01~2 parts by weight, |

wherein said cetirizine hydrochloride is racemic cetirizine hydrochloride, said alcohol having 3 or less carbon atoms is glycerin; said preservative is benzalkonium bromide; and said inorganic salt is sodium chloride.

13. The pharmaceutical solution according to claim 12, **characterized by** comprising the following components:

| Cetirizine hydrochloride | 0.5~1.5 parts by weight; |
|---|---|
| Glycerin | 5-40 parts by weight; |
| Urea | 1~6 parts by weight; |
| Benzalkonium bromide | 0.02~0.15 parts by weight; and |
| Sodium chloride | 0.5~1.5 parts by weight. |

**14.** The pharmaceutical solution according to claim 13, **characterized by** comprising the following components:

| Cetirizine hydrochloride | 1 part by weight; |
|---|---|
| Glycerin | 35-40 parts by weight; |
| Urea | 1~3 parts by weight; |
| Benzalkonium bromide | 0.08~0.12 parts by weight; and |
| Sodium chloride | 0.5~1.5 parts by weight. |

**15.** The pharmaceutical solution according to claim 14, **characterized by** comprising the following components:

| Cetirizine hydrochloride | 1 part by weight; |
|---|---|
| Glycerin | 37.8 parts by weight; |
| Urea | 2 parts by weight; |
| Benzalkonium bromide | 0.1 parts by weight; and |
| Sodium chloride | 0.9 parts by weight. |

**16.** The pharmaceutical solution according to any one of claims 12~15, **characterized in that** the pH of the pharmaceutical solution is 4.0~7.5.

**17.** The pharmaceutical solution according to claim 16, **characterized in that** the pH is 5.0~7.0.

# INTERNATIONAL SEARCH REPORT

| International application No. |
| --- |
| PCT/CN2010/001221 |

**A. CLASSIFICATION OF SUBJECT MATTER**

See extra sheet
According to International Patent Classification (IPC) or to both national classification and IPC

**B. FIELDS SEARCHED**

Minimum documentation searched (classification system followed by classification symbols)

IPC: A61K, A61P

Documentation searched other than minimum documentation to the extent that such documents are included in the fields searched

Electronic data base consulted during the international search (name of data base and, where practicable, search terms used)

EPODOC, WPI, CA, MEDLINE, EMBASE, CPRS, CNKI: Cetirizine, Cetrine, Cistamine, solution, alcohol

**C. DOCUMENTS CONSIDERED TO BE RELEVANT**

| Category* | Citation of document, with indication, where appropriate, of the relevant passages | Relevant to claim No. |
| --- | --- | --- |
| X | CN1957942A (LUNAN PHARM GROUP CORP), 9 May 2007 (09.05.2007), see example 2 | 1-17 |

☐ Further documents are listed in the continuation of Box C.   ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim (S) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 8 Nov. 2010 (08.11.2010) | **18 Nov. 2010 (18.11.2010)** |
| Name and mailing address of the ISA/CN<br>The State Intellectual Property Office, the P.R.China<br>6 Xitucheng Rd., Jimen Bridge, Haidian District, Beijing, China<br>100088<br>Facsimile No. 86-10-62019451 | Authorized officer<br>**LIU,Qiming**<br>Telephone No. (86-10)62411122 |

Form PCT/ISA /210 (second sheet) (July 2009)

**INTERNATIONAL SEARCH REPORT**
Information on patent family members

| | | International application No. |
| --- | --- | --- |
| | | PCT/CN2010/001221 |

| Patent Documents referred in the Report | Publication Date | Patent Family | Publication Date |
| --- | --- | --- | --- |
| CN1957942A | 09.05.2007 | None | |

Form PCT/ISA /210 (patent family annex) (July 2009)

## INTERNATIONAL SEARCH REPORT

International application No.

*PCT/CN2010/001221*

CLASSIFICATION OF SUBJECT MATTER

A61K 31/495 (2006.01) i
A61K 9/08 (2006.01) i
A61P 37/08(2006.01) i
A61P 17/00(2006.01) i

Form PCT/ISA /210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 1634063 A
- CN 1813743 A
- CN 1957942 A

**Non-patent literature cited in the description**

- *Journal for Beneficial Readings: Drug Information & Medical Advices,* 2009, vol. 6, 63
- *JIANGSU Labour Protection,* 2007, vol. 8, 45